# EUROPÄISCHE PATENTSCHRIFT

(11) **EP 1 163 264 B1**
(45) Veröffentlichungstag und Bekanntmachung des Hinweises auf die Patenterteilung: **22.09.2004**
(21) Anmeldenummer: 00910816.8
(22) Anmeldetag: 11.03.2000
(51) Int. Cl.: C07K 7/23, A61K 38/09

(54) **NEUE LHRH-ANTAGONISTEN MIT VERBESSERTEN LÖSLICHKEITSEIGENSCHAFTEN**
NOVEL LHRH ANTAGONISTS WITH IMPROVED SOLUBILITY CHARACTERISTICS
NOUVEAUX ANTAGONISTES DE L'HORMONE DE DECLENCHEMENT DE L'HORMONE LUTEINOTROPHE A PROPRIETES DE DISSOLUTION AMELIOREES

(30) Priorität: 17.03.1999 DE 19911771
(43) Veröffentlichungstag der Anmeldung: 19.12.2001
(73) Patentinhaber: Zentaris GmbH, 60314 Frankfurt/Main (DE)
(72) Erfinder: BERND, Michael, D-60316 Frankfurt (DE); KUTSCHER, Bernhard, D-63477 Maintal (DE); GÜNTHER, Eckhard, D-63477 Maintal (DE); ROMEIS, Peter, D-63571 Gelnhausen (DE); REISSMANN, Thomas, D-60437 Frankfurt (DE); BECKERS, Thomas, D-60596 Frankfurt (DE)
(86) Internationale Anmeldenummer: PCT/EP2000/002165
(87) Internationale Veröffentlichungsnummer: WO 2000/055190

(56) Entgegenhaltungen:
- EP-A- 0 328 090
- EP-A- 0 413 209
- WO-A-97/19953

## Beschreibung

Die Erfindung betrifft LHRH-Antagonisten mit verbesserten Löslichkeitseigenschaften, Verfahren zur Herstellung dieser Verbindungen, Arzneimittel, in denen diese Verbindungen enthalten sind, sowie die Verwendung der Arzneimittel zur Behandlung hormonabhängiger Tumore und hormonbeeinflußter nicht-maligner Erkrankungen wie benigner Prostatahyperplasie (BPH) und Endometriose.

Die zur Definierung der Peptide verwendete Nomenklatur stimmt mit jener durch die IUPAC-IUB-Kommission über Biochemische Nomenklatur erläuterten Nomenklatur überein (European J.Biochem. 1984, 138, 9-37), worin in Übereinstimmung mit der herkömmlichen Darstellung die Aminogruppen beim N-Terminus nach links erscheinen und die Carboxylgruppe beim C-Terminus nach rechts. Die LH-RH-Antagonisten wie die erfindungsgemäßen Peptide umfassen in der Natur vorkommende und synthetische Aminosäuren, wobei erstere Ala, Val, Leu, Ile, Ser, Thr, Lys, Arg, Asp, Asn, Glu, Gln, Cys, Met, Phe, Tyr, Pro, Trp und His umfassen. Die Abkürzungen für die einzelnen Aminosäurereste beruhen auf den Trivialnamen der Aminosäuren und sind Ala=Alanin, Arg=Arginin, Gly=Glycin, Leu=Leucin, Lys=Lysin, Pal(3)=3-(3-Pyridyl)alanin, Nal(2)=3-(2-Naphthyl)alanin, Phe=Phenylalanin, Cpa=4-Chlorphenylalanin, Pro=Prolin, Ser=Serin, Thr=Threonin, Trp=Tryptophan, Tyr=Tyrosin und Sar=Sarkosin. Alle hier beschriebenen Aminosäuren stammen aus der L-Serie, wenn nicht anders erwähnt. Beispielsweise ist D-Nal(2) die Abkürzung für 3-(2-Naphthyl)-D-Alanin und Ser die Abkürzung für L-Serin. Substitutionen an der ε-Aminogruppe in der Seitenkette von Lysin sind durch einen hinter Lys in Klammem gesetzten Ausdruck, gegebenenfalls in Form einer Abkürzung, dargestellt.

Andere verwendete Abkürzungen sind:
- Ac: Acetyl
- Atz: 3-Amino-1,2,4-triazol-5-carbonyl
- B: 4-(4-Amidino-phenyl)-amino-1,4-dioxo-butyl
- Boc: tert. Butyloxycarbonyl
- Bop: Benzotriazol-1-oxy-tris-(dimethylamino)-phosphoniumhexafluorophosphat
- DCC: Dicyclohexylcarbodiimid
- DCM: Dichlormethan
- Ddz: Dimethoxyphenyl-dimethylmethylenoxy-carbonyl (Dimethoxydimethyl-Z)
- DIC: Diisopropylcarbodiimid
- DIPEA: N,N-Diisopropylethylamin
- DMF: Dimethylformamid
- Fmoc: Fluorenylmethyloxycarbonyl
- HF: Flußsäure
- HOBt: 1-Hydroxybenzotriazol
- HPLC: Hochdruckflüssigkeitschromatographie
- Me: Methyl
- TFA: Trifluoressigsäure
- Z: Benzyloxycarbonyl
- Hci: Homocitrullin
- Cpa: 4-Chlorphenylalanin

Die erfindungsgemäßen Peptide stellen Analoge des das luteinisierende Hormon freisetzenden Hormons (LH-RH) dar, das die folgende Struktur aufweist:
p-Glu-His-Trp-Ser-Tyr-Gly-Leu-Arg-Pro-Gly-NH₂, [LH-RH, Gonadorelin].

Während mehr als 20 Jahren haben Forscher nach selektiv potenten Antagonisten des LH-RH-Dekapeptids [M.Karten und J.E.Rivier, Endocrine Reviews 7, 44-66 (1986)] gesucht. Das hohe Interesse an solchen Antagonisten ist in ihrer Nützlichkeit im Bereich der Endokrinologie, Gynäkologie, Schwangerschaftsverhütung und Krebs begründet. Eine große Anzahl von Verbindungen sind als potentielle LH-RH-Antagonisten hergestellt worden. Die interessantesten Verbindungen, die bis heute gefunden wurden, sind jene Verbindungen, deren Strukturen eine Modifizierung der LH-RH-Struktur darstellen.

Die erste Serie von potenten Antagonisten wurde durch die Einführung von aromatischen Aminosäureresten in den Positionen 1, 2, 3 und 6 oder 2, 3 und 6 erhalten. Die übliche Schreibweise der Verbindungen sieht wie folgt aus: Es werden zunächst die Aminosäuren angegeben, die in der Peptidkette von LH-RH an die Stelle der ursprünglich vorhandenen Aminosäuren getreten sind, wobei die Positionen, an denen der Austausch stattfand, durch hochgestellte Ziffern gekennzeichnet werden. Weiterhin wird durch die nachgestellte Bezeichnung "LH-RH" zum Ausdruck gebracht, daß es sich um LH-RH-Analoge handelt, an denen der Austausch stattfand.

Bekannte Antagonisten sind:
[Ac-D-Cpa^{1,2}, D-Trp^{3,6}] LH-RH (D,H.Coy et al., In: Gross, E. and Meienhofer, J. (Eds) Peptides; Proceedings of the 6th American Peptid Symposium, S.775-779, Pierce Chem.Co., Rockville III. (1979):
[Ac-Pro¹, D-Cpa², D-Nal(2)^{3,6}] LH-RH (US-Patent Nr. 4.419.347) und
[Ac- Pro¹, D-Cpa², D-Trp^{3,6}] LH-RH (J.L.Pineda, et al., J. Clin. Endocrinol. Metab. 56, 420, 1983).

Um die Wirkung von Antagonisten zu verbessern, wurden später basische Aminosäuren, zum Beispiel D-Arg, in der 6-Stellung eingeführt. Zum Beispiel [Ac-D-Cpa^{1,2}, D-Trp³, D-Arg⁶, D-Ala¹⁰] LH-RH (ORG-30276) (D.H.Coy, et al., Endocrinology 100, 1445, 1982); und
[Ac-D-Nal(2)1, D-Phe(4-F)², D-Trp³, D-Arg⁶] LH-RH (ORF 1826.0) (J.E. Rivier et al., in: Vickery B.H. Nestor, Jr. J.J., Hafez, E.S.E (Eds). LHRH and its Analogs, S.11-22 MTP Press, Lancaster, UK 1984).

Weitere potente LH-RH-Antagonisten sind in WO 92/19651. WO 94/19370, WO 92/17025, WO 94/14841, WO 94/13313, US-A 5,300,492, US-A 5,140,009, EP 0 413 209 A1 und DE 195 44 212 A1 beschreiben.

Letztere offenbart Verbindungen mit einem modifizierten Omithin- oder Lysin-Baustein in Position 6, welche der folgenden Formel entsprechen:
Ac-D-Nal(2)¹-D-Cpa²-D-Pal(3)³-Ser⁴-Tyr⁵-D-Xxx⁶-Leu⁷-Arg⁸-Pro⁹-D-Ala¹⁰-NH₂,
worin D-Xxx eine Aminosäuregruppe der allgemeinen Formel (VI) 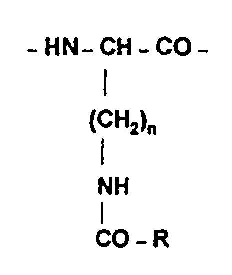 darstellt.

LH-RH-Antagonisten sind weiterhin in der WO 97/19953 und der EP-A2 0 328 090 beschrieben.

Weitere bekannte LH-RH-Antagonisten sind Antarelix, Ganirelix und Cetrorelix.

### Antarelix:

Ac-D-Nat(2)¹-D-Cpa²-D-Pal(3)³-Ser⁴-Tyr⁵-D-Hci⁶-Leu⁷ -Lys(iPr)⁸-Pro⁹-D-Ala¹⁰-NH₂

### Ganirelix:

Ac-D-Nal(2)¹-D-Cpa² -D-Pal(3)³ -Ser⁴- Tyr⁵ -D-hArg(Et)₂⁶-Leu⁷-hArg(Et)₂⁸ -Pro⁹-D-Ala¹⁰-NH₂

### Cetrorelix:

Ac-D-Nal(2)¹-D-Cpa²-D-Pal(3)³-Ser⁴-Tyr⁵-D-Cit⁶-Leu⁷-Arg⁸-Pro⁹-D-Ala¹⁰-NH₂ .

Ziel der Erfindung ist, neue LH-RH-Antagonisten zu schaffen, die eine erhöhte enzymatische Stabilität und signifikant verbesserte Wasserlöslichkeit aufweisen.

Diese Aufgabe wird durch Verbindungen der folgenden allgemeinen Formel (I) gelöst,

A-Xxx¹-Xxx²-Xxx³-Xxx⁴-Xxx⁵- Xxx⁶ Xxx⁷-Xxx⁸-Xxx⁹-Xxx¹⁰-NH₂ (I)

worin
A eine Acetyl- oder eine 3-(4-Fluorphenyl)-propionyl-Gruppe,
Xxx¹ D-Nal(1) oder D-Nal(2),
Xxx²-Xxx³ D-Cpa-D-Pal(3) oder eine Einfachbindung,
Xxx⁴ Ser,
Xxx⁵ N-Me-Tyr,
Xxx⁶ D-Hci
Xxx⁷ Nle,
Xxx⁸ Arg oder Lys(iPr),
Xxx⁹ Pro und
Xxx¹⁰ Ala oder Sar bedeutet,
und deren Salze mit pharmazeutisch akzeptablen Säuren, insbesondere die Acetate, Embonate und Trifluoracetate.

Besonders bevorzugte erfindungsgemäße Verbindungen sind:
Ac-D-Nal(2)¹-D-Cpa²-D-Pal(3)³-Ser⁴-N-Me-Tyr⁵-D-Hci⁶-Nle⁷-Arg⁸-Pro⁹-D-Ala¹⁰-NH₂,
Ac-D-Nal(2)¹-D-Cpa²-D-Pal(3)³-Ser⁴-N-Me-Tyr⁵-D-Hci⁶-Nle⁷-Lys(iPr)⁸-Pro⁹-Ala¹⁰-NH₂,
Ac-D-Nal(2)¹-D-Cpa²-D-Pal(3)³-Ser⁴-N-Me-Tyr⁵-D-Hci⁶-Nle⁷-Lys(iPr)⁸-Pro⁹-Sar¹⁰-NH₂,
Ac-D-Nal(2)¹-D-Cpa²-D-Pal(3)³-Ser⁴-N-Me-Tyr⁵-D-Hci⁶ -Nle⁷-Arg⁸-Pro⁹-Sar¹⁰-NH₂, sowie deren Salze mit den obengenannten pharmazeutisch akzeptablen Säuren.

Die erfindungsgemäßen Verbindungen können zur Behandlung hormonabhängiger Tumore, insbesondere Prostatacarcinom oder Brustkrebs, sowie für nicht-maligne Indikationen, deren Behandlung eine LH-RH-Hormonsuppression erfordert, verwendet werden. Dazu werden sie mit den üblichen Träger- und Hilfsstoffen vermischt und als Arzneimittel konfektioniert.

Die Synthese von Verbindungen gemäß Formel (I) kann sowohl entweder durch klassische Fragmentkondensation oder per Festphasensynthese nach Merrifield mit aufeinander abfolgendem Aufbau unter Verwendung von in der Seitenkette bereits mit der Carbonsäure der allgemeinen Formel R¹-COOH acyliertem D-Lysin als auch durch Umsetzung eines Decapeptidbausteins mit den entsprechenden Carbonsäuren durch Amid-Verknüpfung in der Seitenkette von D-Lysin⁶ erfolgen. Demnach kann die Einführung der R¹-CO-Gruppe an drei verschiedenen Stellen des Verfahrens vorgenommen werden: vor der Kondensation der Einzelbausteine zum Peptid, nach dem Einbau von Lysin oder Omithin in der Peptidkette, aber vor der Kondensation des nächstfolgenden Bausteins oder nach Kondensation aller Bausteine.

Die Verbindungen der Formel (I) werden nach den bekannten Methoden synthetisiert, wie zum Beispiel durch reine Festphasentechnik, teilweise Festphasentechnik (sogenannte Fragmentkondensation) oder durch die klassischen Lösungskupplungen (siehe M.Bodanszky, "Principles of Peptide Synthesis". Springer Verlag 1984).

Zum Beispiel sind die Methoden der Festphasensynthese im Lehrbuch "Solid Phase Peptide Synthesis" J.M.Stewart and J.D.Young, Pierce Chem.Company, Rockford, III, 1984, und in G.Barany and R.B.Merrifield 'The Peptides", Ch.1, S.1-285, 9979, Academic Press Inc. beschrieben. Klassische Lösungssynthesen sind ausführlich in der Behandlung "Methoden der Organischen Chemie (Houben-Weyl), Synthese von Peptiden" E.Wünsch (Herausgeber) 1974, Georg Thieme Verlag, Stuttgart, BRD, beschrieben.

Der stufenweise Aufbau erfolgt zum Beispiel, indem man zunächst die Carboxy-terminale Aminosäure, deren α-ständige Aminogruppe geschützt ist, an einen hierfür üblichen unlöslichen Träger kovalent bindet, die α-Amino-Schutzgruppe dieser Aminosäure abspaltet, an die so erhaltene freie Aminogruppe die nächste geschützte Aminosäure über ihre Carboxy-Gruppe bindet, und in dieser Weise Schritt für Schritt die übrigen Aminosäuren des zu synthetisierenden Peptids in der richtigen Reihenfolge verknüpft, und nach Verknüpfung aller Aminosäuren das fertige Peptid vom Träger abspaltet und gegebenenfalls weitere vorhandene Seitenfunktions-Schutzgruppen abspaltet Die stufenweise Kondensation erfolgt durch Synthese aus den entsprechenden, in üblicher Weise geschützten Aminosäuren in herkömmlicher Weise.

Die Verknüpfung der einzelnen Aminosäuren miteinander erfolgt nach den hierfür üblichen Methoden, insbesondere kommen in Frage:
- Methode der symmetrischen Anhydride in Gegenwart von Dicyclohexylcarbodiimid oder Diisopropylcarbodiimid (DCC, DIC)
- Carbodiimid-Methode allgemein
- Carbodiimid-Hydroxybenzotriazol-Methode
(siehe The Peptides, Volume 2, Ed. E. Gross and J. Meienhofer).

Bei der Fragmentkupplung verwendet man vorzugsweise die ohne Racemisierung verlaufende Azidkupplung oder die DCC-1-Hydroxybenzotriazol- beziehungsweise DCC-3-Hydroxy-4-oxo-3,4-dihydro-1,2,3-benzotriazin-Methode. Man kann auch aktivierte Ester von Fragmenten einsetzen.

Für die stufenweise Kondensation von Aminosäuren eignen sich besonders gut aktivierte Ester von N-geschützten Aminosäuren, wie zum Beispiel N-Hydroxysuccinimidester oder 2,4,5-Trichlorphenylester. Die Aminolyse läßt sich sehr gut durch N-Hydroxyverbindungen, die in etwa die Acidität der Essigsäure besitzen, wie zum Beispiel 1-Hydroxybenzotriazol, katalysieren.

Als intermediäre Aminoschutzgruppen bieten sich abhydrierende Gruppen, wie zum Beispiel der Benzyloxycarbonylrest (= Z-Rest) oder schwach sauer abspaltbare Gruppen an. Als Schutzgruppen für die α-ständigen Aminogruppen kommen zum Beispiel in Frage:
tertiäre Butyloxycarbonylgruppen, Fluorenylmethyloxycarbonylgruppen Carbobenzoxygruppen beziehungsweise Carbobenzthiogruppen (gegebenenfalls jeweils mit p-Brom oder p-Nitro-benzylrest), die Trifluoracetylgruppe, der Phthalylrest, die o-Nitrophenoxyacetylgryppe, die Tritylgruppe, die p-Toluolsulfonylgruppe, die Benzylgruppe, im Benzolkern substituierte Benzylreste (p-Brom oder p-Nitro-benzylrest) und der α-Phenylethylrest. Hierzu wird auch auf Jesse P. Greenstein und Milton Winitz, Chemistry of Amino Acids, New York 1961, John Wiley and Sons, Inc., Volume 2, beispielsweise Seite 883 und folgende, "Principles of Peptide Synthesis", Springer Verlag 1984, "Solid Phase Peptide Synthesis" J.M.Stewart and J.D.Young, Pierce Chem.Company, Rockford, III, 1984, G.Barany and R.B.Merrifietd "The Peptides", Ch.1, S.1-285, 1979, Academic Press Inc. sowie The Peptides, Volume 2, Ed.E. Gross and J. Maienhofer, Academic Press, New York, verwiesen. Diese Schutzgruppen kommen grundsätzlich auch für den Schutz von weiteren funktionellen Seitengruppen (OH-Gruppen, NH₂-Gruppen) der entsprechenden Aminosäuren in Frage.

Vorhandene Hydroxygruppen (Serin, Threonin) werden vorzugsweise durch Benzylgruppen und ähnliche Gruppen geschützt. Weitere nicht α-ständige Aminogruppen (zum Beispiel Aminogruppen in ω-Stellung, Guanidinogruppe des Arginins) werden vorzugsweise orthogonal geschützt.

Die einzelnen Aminosäurebausteine sind, ausgenommen durch die R¹-CO-Gruppe modifiziertes Lysin oder Omithin, käuflich erhältlich. Ein möglicher Ablauf des Verfahrens zur Herstellung der letzteren Verbindungen ist wie folgt:
1. Die α-Carbonsäuregruppe wird amidiert.
2. Die ε-Aminogruppe wird mit der Z-Gruppe geschützt.
3. Die α-Aminogruppe wird mit der Boc-Gruppe geschützt, so daß sich eine Selektivität bezüglich der späteren Abspaltung der Aminoschutzgruppen ergibt.
4. Die Z-Gruppe an der ε-Aminogruppe wird abgespalten.
5. An der ε-Aminogruppe wird die gewünschte Gruppe R⁴-CO- eingeführt.
6. Die Boc-Gruppe an der α-Aminogruppe wird abgespalten.
7. Die α-Aminogruppe wird mit der Z-Gruppe versehen.

Für die Einführung der R¹-CO-Gruppe durch Umsetzen der Aminogruppe des Lysins mit der entsprechenden Carbonsäure kommen grundsätzlich die gleichen Vefahren wie oben für die Verknüpfung der Aminosäuren beschriebenen in Frage. Besonders bevorzugt ist jedoch die Kondensation unter Verwendung von Carbodiimid, beispielsweise 1-Ethyl-3-(3-dimethylaminopropyl)-carbodiimid, und 1-Hydroxybenzotriazol.

Die Reaktion zur Verknüpfung vom Aminosäuren findet in einem hierfür üblichen indifferenten Lösungs- oder Suspensionsmittel (zum Beispiel Dichlormethan) statt, wobei gegebenenfalls zur Verbesserung der Löslichkeit Dimethylformamid zugesetzt werden kann.

Als synthetisches Trägermaterial kommen unlösliche Polymere in Frage, zum Beispiel in organischen Lösungsmittel quellbares Polystyrolharz in Perlenform (beispielsweise ein Copolymerisat aus Polystyrol und 1% Divinylbenzol). Der Aufbau eines geschützten Decapeptidamids an einem Methyl-benzhydrylamid-Harz (MBHA-Harz, d.h. mit Methyl-benzhydrylamid-Gruppen versehenes Polystyrolharz), welches die gewünschte C-terminale Amidfunktion des Peptids nach HF-Spaltung vom Träger ergibt, kann gemäß folgendem Fließdiagramm durchgeführt werden:

| Fließdiagramm | | | |
|---|---|---|---|
| Peptid-Syntheseprotokoll | | | |
| Stufe | Funktion | Lösungsmittel/Reagenz (v/v) | Zeit |
| 1 | Waschen | Methanol | 2 x 2 min |
| 2 | Waschen | DCM | 3 x 3 min |
| 3 | Abspaltung | DCM/TFA (1:1) | 1 x 30 min |
| 4 | Waschen | Isopropanol | 2 x 2 min |
| 5 | Waschen | Methanol | 2 x 2 min |
| 6 | Waschen | DCM | 2 x 3 min |
| 7 | Neutralisation | DCM/DIPEA (9:1) | 3 x 5 min |
| 8 | Waschen | Methanol | 2 x 2 min |
| 9 | Waschen | DCM | 3 x 3 min |
| 10 | STOP | Zugabe der Boc-As in DCM + DIC + HOBt | |
| 11 | Kupplung | DCM, ggf. DCM/DCF | ca. 90 min |
| 12 | Waschen | Methanol | 3 x 2 min |
| 13 | Waschen | DCM | 2 x 3 min |

Die Nα-Boc-geschützten Aminosäuren werden üblicherweise in dreifachem molaren Überschuß in Gegenwart von Diisopropylcarbodiimid (DIC) und 1-Hydroxybenzotriazol (HOBt) in CH₂Cl₂/DMF innerhalb von 90 min. gekuppelt. und die Boc-Schutzgruppe durch halbstündige Einwirkung von 50% Trifluoressigsäure (TFA) in CH₂Cl₂ abgespalten. Zur Kontrolle des vollständigen Umsatzes kann der Chloraniltest nach Christensen und der Kaiser'sche Ninhydrintest dienen. Reste freier Aminofunktion werden durch Acetylierung in fünffachem Überschuß an Acetylimidazol in CH₂Cl₂ blockiert. Die Abfolge der Reaktionsschritte des Peptidaufbaus am Harz ergibt sich aus dem Fließdiagramm. Zur Abspaltung der harzgebundenen Peptide wird das jeweilige Endprodukt der Festphasensynthese im Vakuum über P₂O₅ getrocknet und in 500fachem Überschuß an HF/Anisol 10:1/V:V 60 min. bei 0°C behandelt.

Nach Abdestillation von HF und Anisol im Vakuum fallen die Peptidamide durch Ausrühren mit wasserfreiem Ethylether als weiße Feststoffe an, die Abtrennung von mit anfallendem polymeren Träger erfolgt durch Auswaschen mit 50%iger wäßriger Essigsäure. Durch schonendes Einengen der essigsauren Lösungen im Vakuum können die jeweiligen Peptide als hochviskose Öle erhalten werden, welche sich nach Zugabe von abs.Ether in der Kälte in weiße Feststoffe umwandeln.

Die weitere Aufreinigung erfolgt durch Routinemethoden der präparativen Hochdruck-Flüssigskeitschromatographie (HPLC).

Das Überführen der Peptide in ihre Säureadditionssalze kann durch Umsetzen derselben mit Säuren in an sich bekannter Weise bewerkstelligt werden. Umgekehrt können freien Peptide durch Umsetzen ihrer Säureadditionssalze mit Basen erhalten werden. Peptidembonate können durch Umsetzung von Trifluoressigsäuresalzen (TFA-Salzen) des Peptids mit freier Embonsäure (Pamoasäure) oder dem entsprechenden Dinatrium-Salz der Embonsäure dargestellt werden. Dazu wird das Peptid-TFA-Salz in wäßriger Lösung mit der Lösung von Dinatrium-embonat in polar-aprotischem Medium, bevorzugt Dimethylacetamid, versetzt und der sich bildende hellgelbe Niederschlag isoliert.

Die folgenden Beispiele dienen der Erläuterung der Erfindung, ohne diese zu beschränken.

### Beispiel 1

### Ac-D-Nal(2)¹-D-Cpa²-D-Pal(3)³-Ser⁴-N-Me-Tyr⁵-D-Hci⁶-Nle⁷-Arg⁸-Pro⁹-D-Ala¹⁰-NH₂

Die Synthese erfolgte gemäß Festphasen-Fließdiagramm (Peptidsynthese-Protokoll, S. 11) mit DIC/HOBt-Kupplung, ausgehend von 3.3 g MBHA-Harz (Beladungsdichte 1.08 mmol/g). Nach HF-Abspaltung vom polymeren Träger fielen 3.4 g Rohpeptid an, welches durch Standardverfahren der präparativen HPCI aufgereinigt wurden. Nach anschließender Gefriertrocknung erhielt man 1.43 g HPLC-einheitliches Produkt der Summenformel C72, H96, N17, O14, Cl mit korrektem FAB-MS: 1458.7 (M+H⁺) (ber: 1457.7), und entsprechendem ¹H-NMR-Spektrum.
¹H-NMR (500 MHz, D₂O/DMSO-d₆, δ in ppm):
8,7 bis 7.2, mehrere m, arom. H und nicht vollständig ausgetauschte NH; 6.92 u. 6.58, 2d, 2x2H, arom.H p-Cl-Phe; 5.2 bis 3.5, mehrere m, Cα-H u. aliph.H; 3.2 bis 2.6, mehrere m, aromat. Cβ-H, 2.1 bis 0.7, mehrere m, restl. aliphat. H; 1,70, s, 3H, Acetyl; 1,20, d, 3H, Cβ-H Ala; 0,8, m, Cδ-H Leu

### Beispiel 2

### Ac-D-Nal(2)¹-D-Cpa²-D-Pal(3)³-Ser⁴-N-Me-Tyr⁵-D-Hci⁶-Nle⁷-Lys(iPr)⁸-Pro⁹-Ala¹⁰-NH₂

Die Synthese erfolgte gemäß Festphasen-Fließdiagramm (Peptidsynthese-Protokoll, S. 11) mit DIC/HOBt-Kupplung, ausgehend von 2.5 g MBHA-Harz (Beladungsdichte 1.08 mmol/g). Nach HF-Abspaltung vom polymeren Träger fielen 2.78 g Rohpeptid an, welches durch Standardverfahren der präparativen HPCI aufgereinigt wurden. Nach anschließender Gefriertrocknung erhielt man 400 mg HPLC-einheitliches Produkt der Summenformel C75, H102, N15, O14, Cl mit korrektem ESI-MS: 1472.6 (M+H⁺) (ber: 1471.7) und entsprechendem ¹H-NMR-Spektrum.
¹H-NMR (500 MHz, D₂O/DMSO-d₆, δ in ppm):
8.62, m, 2H, 8.30, m, 2H,7.80, m, 4H, 7.66, s, 1H, 7.47, m, 2H, 7.36, d, 1H, aromat. H; 7.25 und 7.20, 2 d, 4H, arom.H (pCl)Phe; 6.96 und 6.63, 2 d, 4H, aromat. H Tyr, 5.10 bis 4.0, mehrere m, Cα-H und aliphat H; 3.75 bis 2.65, mehrere m, Cβ-H und N-CH₃; 2.1 bis 1.05, mehrere m, restl. aliphat H; 1.74, s, 3H, Acetyl; 1,23, d, 3H, Cβ-H Ala; 1.20, m, CH₃ Isoprop. ;0,8, m, 3H, Cδ-H Nle

### Beispiel 3

### Ac-D-Nal(2)¹-D-Cpa²-D-Pa!(3)³-Ser⁴-N-Me-Tyr⁵-D-Hci⁶-Nle⁷-Lys(iPr)⁸-Pro⁹-Sar¹⁰-NH₂

Die Synthese erfolgte gemäß Festphasen-Fließdiagramm (Peptidsynthese-Protokoll, S. 11) mit DIC/HOBt-Kupplung, ausgehend von 2.5 g MBHA-Harz (Beladungsdichte 1.08 mmol/g). Nach HF-Abspaltung vom polymeren Träger fielen 2.74 g Rohpeptid an, welches durch Standardverfahren der präparativen HPCI aufgereinigt wurden. Nach anschließender Gefriertrocknung erhielt man 840 mg HPLC-einheitliches Produkt der Summenformel C75, H102, N15, O14, Cl mit korrektem ESI-MS: 1472.6 (M+H⁺) (ber. 1471.7) und entsprechendem ¹H-NMR-Spektrum.
¹H-NMR (500 MHz, D₂O/DMSO-d₆, δ in ppm):
8.6, m, 2H, 8.3, m, 2H,7.85, m, 2H, 7.8, m, 2H, 7.65, s, 1H, 7.46, m, 2H, 7.35, d, 1H, aromat. H; 7.23 und 7.17, 2 d, 4H, arom.H (pCl)Phe; 7.0 und 6.6, 2 d, 4H, aromat. H Tyr; 5.10 bis 3.8, mehrere m, Cα-H und aliphat H; 3.75 bis 2.6, mehrere m, Cβ-H und N-CH₃; 2.2 bis 1.05, mehrere m, restl. aliphat H; 1.70, s, 3H, Acetyl; 1,23, d. 3H, Cβ Ala; 1.20, m, CH₃ Isoprop. ;0,8, m, 3H, Cδ Nle

Die erfindungsgemäßen Verbindungen nach Formel 1 wurden auf ihre Rezeptorbindung untersucht. Das Verfahren lehnte sich eng an das in Beckers et al., Eur. J. Biochem. 231, 535-543 (1995), beschriebene Verfahren an. Nach der oben offenbarten Synthese erhaltenes Cetrorelix wurde mit [¹²⁵l] (Amersham; spezifische Aktivität 80.5Bq/fmol) unter Verwendung des IodoGen-Reagens (Pierce) iodiert. Das Reaktionsgemisch wurde durch Umkehrphasen-Hochleistungs-Flüssigkeitschromatographie gereinigt, wobei mono-iodiertes Cetrorelix ohne unmarkiertes Peptid erhalten wurde. Jeweils etwa 80% des [¹²⁵I]-Cetrorelix und der nicht markierten erfindungsgemäßen Verbindung waren zur spezifischen Rezeptorassoziation geeignet.

Die erfindungsgemäßen Verbindungen können mit den folgenden Methoden 1 und 2 auf ihre In-vitro-Wirkung getestet werden, wobei die Bindungsaffinitäten im Bindungsassay mit [¹²⁵l]-Cetrorelix (Methode 1) und die funktionalen Aktivitäten mit Triptorelin als agonistischem Stimulus (Methode 2) bestimmt wurden.

### Methode 1.

Rezeptorbindungs-Assay nach Beckers, T., Marheineke, K, Reiländer, H., Hilgard P. (1995) "Selection and characterization of mammalian cell lines with stable overexpression of human pituitary receptors for gonadoliberin (GnRH)" Eur. J. Biochem. 231, 535 - 543.

Zur Untersuchung der Rezeptorbindung wurde Cetrorelix unter Verwendung des lodoGen-Reagens (Pierce) mit [¹²⁵I] (Amersham; 80.5Bq/fmol spezifische Aktivität) iodiert. Das Reaktionsgemisch wurde durch Hochleistungs-Flüssigkeitschromatographie mit vertauschten Phasen gereinigt, wobei mono-iodiertes Cetrorelix ohne unmarkiertes Peptid erhalten wurde. Etwa 80% des [¹²⁵l] Cetrorelix war zur spezifischen Rezeptorassoziation befähigt.

Der Rezeptorbindungs-Assay wurde mit intakten Zellen unter physiologischen Bedingungen wie beschrieben (Beckers et al. 1995) durchgeführt. Subkonfluente Kulturen von stabil transfizierten LTK -Zellen, die den humanen LHRH-Rezeptor exprimieren, wurden durch Inkubation in NaCl/Pᵢ (137mM NaCl, 2.7mM KCl, 8.1 mM Na₂HPO₄, 11.47mM KH₂PO₄)/ 1 mM EDTA abgetrennt und durch Zentrifugieren gesammelt. Das Zellpellet wurde in Bindungspuffer (DMEM ohne H₂CO₃, mit4.5g/l glucose, 10mM Hepes pH7.5, 0.5% (Masse/Volumen) BSA, 1g/l Bacitracin, 0.1g/l SBTI, 0.1% (Masse/Volumen) NaN₃) resuspendiert. Für Verdrängungs-Assays wurden 0.25x10⁶ Zellen/100µl mit etwa 225pM des [¹²⁵I]-Cetrorelix (spezifische Aktivität 5 -10 x10⁵ dpm/pmol) und verschiedenen Konzentrationen von unmarkierter erfindungsgemäßer Verbindung als Kompetitor inkubiert. Die Zellsuspension in 100µl Bindungsmedium wurde in 400µl Assayröhrchen über 200µl 84 Vol.-% Siliconöl (Merck Typ 550) /16 Vol.-% Paraffinöl geschichtet. Nach Inkubation für 1 h bei 37°C unter langsamem, kontinuierlichem Schütteln wurden die Zellen durch Zentrifugieren für 2min bei 9000rpm (Rotortyp HTA13.8; Heraeus Sepatec, Osterode/Germany) von dem Inkubationsmedium getrennt. Die Spitzen der Röhrchen, die das Zellpellet enthielten, wurden abgeschnitten. Zellpellet und Überstand wurden anschließend durch Zählung der γ-Strahlung analysiert. Die Menge an unspezifisch Gebundenem wurde unter Einschluß von unmarkiertem Cetrorelix bei 1µM Endkonzentration bestimmt und betrug typischerweise ≤ 10% des gesamten Gebundenen. Die Analyse der Bindungsdaten wurde mit dem EBDA/Ligand-Analyseprogramm (Biosoft V3.0) durchgeführt.

### Methode 2.

### Funktioneller Assay zur Bestimmung der antagonistischen Wirksamkeit

Der Assay wurde mit einigen Modifizierungen versehen so durchgeführt wie in Beckers, T., Reiländer, H., Hilgard, P. (1997) "Characterization of gonadotropin-releasing hormone analogs based on a sensitive cellular luciferase reporter gene assay", Analyt. Biochem. 251, 17 - 23 (Beckers et al. 1997) beschrieben. 10.000 Zellen pro Vertiefung, die den humanen LHRH-Rezeptor und ein Luciferase-Reportergen exprimieren, wurden 24h in Mikrotiterplatten unter Verwendung von DMEM mit Zusätzen und 1 % (v:v) FCSᵢ kultiviert. Die Zellen wurden anschließend 6h mit 1 nM [D-Trp⁶] LHRH stimuliert Antagonistische erfindungsgemäße Verbindungen wurden vor der Stimulierung zugegeben und die Zellen wurden zum Schluß zur Quantifizierung der zellulären Luc-Activität lysiert. Die Berechnung der IC₅₀-Werte aus Dosis-Wirkungs-Kurven wurde durch nicht-lineare Regressionsanalyse unter Verwendung des Hill-Modells (Programm EDX 2.0 von C. Grunwald, Arzneimittelwerk Dresden) durchgeführt.

Die Quantifizierung der Luc-Aktivität wurde im wesentlichen wie beschrieben (Promega Technical Bulletins #101/161) unter Verwendung des jeweiligen Luciferase-Assaysystems (Promega E4030) in Duplikaten durchgeführt. Durch Zugabe von Coenzyme A (CoA) findet eine Oxydation von Luciferyl-CoA mit vorteilhafter Kinetik statt. Nach der Entfernung des Kulturmediums von der Mikrotiterplatte wurden die Zellen durch Zugabe von 100µl Lysepuffer (25mM Tris-phosphate pH7.8, 2mM Dithiothreitol, 2mM 1,2-diaminocyclohexan-N,N,N',N'-tetra-essigsäure (CDTA), 10% (v:v) Glycerin, 1% (v:v) Triton X-100) lysiert. Nach 15min Inkubation bei Raumtemperatur wurden 10µl Zelllysat in eine für luminometrische Detektion geeignete weiße Mikrotiterplatte (Dynatech) überführt. Die enzymatische Reaktion wurde durch Zugabe von 50µl Assaypuffer (20mM Tricin pH7.8, 1.07mM (MgCO₃)₄Mg(OH)₂, 2.67mM MgSO₄, 0.1 mM Ethylenediamin-tetraessigsäure (EDTA), 33.3mM Dithiothreitol, 270µM Coenzym A, 470µM Glühwürmchen(Photinus pyralis)-Luciferin, 530µM rATPNa₂) initiiert. Nach einer Minute wurde für eine Gesamtzeit von einer Sekunde die Lumineszenz mit einer Signalhalbwertszeit von fünf Minuten unter Verwendung des EG&G Berthold MicroLumat LB 96 P bestimmt.

Auf diese Weise wurden folgende In-vitro-Daten erhalten, wobei K_{D} für die Bindungsaffinitäten und IC₅₀ für die funktionale Aktivität steht und pM Pikomol pro Liter bedeutet:

| Verbindung | K_{D} [pM] | IC₅₀ [pM] |
|---|---|---|
| Cetrorelix | 170(21) | 198 (5) |
| Beispiel 1 (Acetat Salz) | n. b. | 242 (3) |
| n. b. = nicht bestimmt | | |
| ( ) = Anzahl der voneinander unabhängigen Versuche | | |

## Patentansprüche

1. Verbindungen der allgemeinen Formel 1
A-Xxx¹-Xxx²-Xxx³-Xxx⁴-Xxx⁵ Xxx⁶-Xxx⁷-Xxx⁸-Xxx⁹-Xxx¹⁰-NH₂ (I)
worin
A eine Acetyl- oder eine 3-(4-Fluorphenyl)-propionyl-Gruppe,
Xxx¹ D-Nal(1) oder D-Nal(2),
Xxx²-Xxx³ D-Cpa-D-Pal(3) oder eine Einfachbindung,
Xxx⁴ Ser,
Xxx⁵ N-Me-Tyr,
Xxx⁶ D-Hci
Xxx⁷ Nle,
Xxx⁸ Arg oder Lys(iPr),
Xxx⁹ Pro und
Xxx¹⁰ Ala oder Sar bedeutet,
und deren Salze mit pharmazeutisch akzeptablen Säuren.

2. Verbindungen nach Anspruch 1, worin das Salz ein Acetat, Trifluoracetat, oder Embonat ist.

3. Verbindung nach Anspruch 1 mit der Formel:
Ac-D-Nal(2)¹-D-Cpa²-D-Pal(3)³-Ser⁴-N-Me-Tyr⁵-D-Hci⁶-Nle⁷-Arg⁸-Pro⁹-D-Ala¹⁰-NH₂.

4. Verbindung nach Anspruch 1 mit der Formel:
Ac-D-Nal(2)¹-D-Cpa²-D-Pal(3)³-Ser⁴-N-Me-Tyr⁵-D-Hci⁶-Nle⁷-Lys(iPr)⁸-Pro⁹-Ala¹⁰-NH₂.

5. Verbindung nach Anspruch 1 mit der Formel:
Ac-D-Nal(2)¹-D-Cpa²-D-Pal(3)³-Ser⁴-N-Me-Tyr⁵-D-Hci⁶-Nle⁷-Lys(iPr)⁸-Pro⁹-Sar¹⁰-NH₂.

6. Verbindung nach Anspruch 1 mit der Formel: Ac-D-Nal(2)¹-D-Cpa²-D-Pal(3)³-Ser⁴-N-Me-Tyr⁵-D-Hci⁶-Nle⁷-Arg⁸-Pro⁹-Sar¹⁰-NH₂.

7. Pharmazeutische Zusammensetzung, umfassend eine Verbindung nach einem der Ansprüche 1 bis 6.

8. Verfahren zur Herstellung von Verbindungen der allgemeinen Formel I nach Anspruch 1, bei dem Fragmente aus mit geeigneten Schutzgruppen versehenen Bausteinen Xxx^{m}, bei denen m eine ganze Zahl von 1 bis 10 bedeutet und Xxx¹ acetyliert ist, an einer Festphase oder in Lösung nach üblichen Verfahren aufgebaut werden, anschließend die Fragmente an einer Festphase durch Segmentkupplung verbunden werden und nach Abschluß der Kupplung die Verbindungen der allgemeinen Formel I mit üblichen Verfahren unter Amidierung am Baustein Xxx¹⁰ von der Festphase abgespalten werden.

9. Verwendung der Substanzen gemäß den Ansprüchen 1 bis 6 zur Herstellung von Arzneimitteln zur Behandlung hormonabhängiger Tumore, insbesondere Prostatacarcinom oder Brustkrebs, sowie für nicht-maligne Indikationen, deren Behandlung eine LH-RH-Hormonsuppression erfordert.

10. Verfahren zur Herstellung von Arzneimitteln, bei dem man Verbindungen gemäß den Ansprüchen 1 bis 6 mit den üblichen Träger- und Hilfsstoffen vermischt und als Arzneimittel konfektioniert.

## Claims

1. Compounds of the general formula I
A-Xxx¹-Xxx²-Xxx³-Xxx⁴-Xxx⁵-Xxx⁶-Xxx⁷-Xxx⁸-Xxx⁹-Xxx¹⁰-NH₂ (I)
in which
A is an acetyl or a 3-(4-fluorophenyl)propionyl group,
Xxx¹ is D-Nal(1) or D=Nal(2),
Xxx²-Xxx³ is D-Cpa-D-Pal(3) or a single bond,
Xxx⁴ is Ser,
Xxx⁵ is N-Me-Tyr,
Xxx⁶ is D-Hci,
Xxx⁷ is Nle,
Xxx⁸ is Arg or Lys(iPr),
Xxx⁹ is Pro and
Xxx¹⁰ is Ala or Sar,
and their salts with pharmaceutically acceptable acids.

2. Compounds according to Claim 1, in which the salt is an acetate, trifluoroacetate or embonate.

3. Compound according to Claim 1 having the formula:
Ac-D-Nal(2)¹-D-Cpa²-D-Pal(3)³-Ser⁴-N-Me-Tyr⁵-D-Hci⁶-Nle⁷-Arg⁸-Pro⁹-D-Ala¹⁰-NH₂.

4. Compound according to Claim 1 having the formula:
Ac-D-Nal (2)¹-D-Cpa²-D-Pal (3)³-Ser⁴-N-Me-Tyr⁵-D-Hci⁶-Nle⁷-Lys(iPr)⁸-Pro⁹-D-Ala¹⁰-NH₂.

5. Compound according to Claim 1 having the formula:
Ac-D-Nal (2)¹-D-Cpa² -D-Pal (3)³-Ser⁴-N-Me-Tyr⁵-D-Hci⁶-Nle⁷-Lys (iPr)⁸-Pro⁹-Sar¹⁰-NH₂.

6. Compound according to Claim 1 having the formula:
Ac-D-Nal (2)¹-D-Cpa²-D-Pal(3)³-Ser⁴ -N-Me-Tyr⁵-D-Hci⁶-Nle⁷-Arg⁸-Pro⁹-Sar¹⁰-NH₂.

7. Pharmaceutical composition comprising a compound according to one of Claims 1 to 6.

8. Process for the preparation of compounds of the general formula I according to Claim 1, in which fragments from units Xxx^{m} provided with suitable protective groups, in which m is an integer from 1 to 10 and Xxx¹ is acetylated, are synthesized on a solid phase or in solution according to customary processes, then the fragments are linked to a solid phase by segment coupling and after conclusion of the coupling the compounds of the general formula I are removed from the solid phase using customary processes with amidation on the unit Xxx¹⁰.

9. Use of the substances according to Claims 1 to 6 for producing medicaments for the treatment of hormone-dependent tumours, in particular prostate carcinoma or breast cancer, and also for non-malignant indications whose treatment necessitates LH-RH hormone suppression.

10. Process for producing medicaments, in which compounds according to Claims 1 to 6 are mixed with the customary vehicles and excipients and formulated as medicaments.

## Revendications

1. Composés répondant à la formule générale I
A-Xxx¹, Xxx², Xxx³, Xxx⁴, Xxx⁵, Xxx⁶, Xxx⁷, Xxx⁸, Xxx⁹, Xxx¹⁰ -NH₂ (I)
dans laquelle :
A représente un groupe acétyle ou un groupe 3-(4-fluorophényl)-propionyle,
Xxx¹ représente D-Nal(1) ou D-Nal(2),
Xxx²- Xxx³ représente D-Cpa-D-Pal(3) ou une liaison simple,
Xxx⁴représente Ser,
Xxx⁵ représente N-Me-Tyr,
Xxx⁶ représente D-Hci,
Xxx⁷ représente Nle,
Xxx⁸ représente Arg ou Lys(iPr),
Xxx⁹ représente Pro et
Xxx¹⁰ représente Ala ou Sar,
et leurs sels avec des acides pharmaceutiquement acceptables.

2. Composés selon la revendication 1, dans lesquels le sel est un acétate, un trifluoroacétate ou un embonate.

3. Composé selon la revendication 1 répondant à la formule :
Ac-D-Nal(2)¹-D-Cpa²-D-Pal(3)³-Ser⁴-N-Me-Tyr⁵-D-Hci⁶-Nle⁷-Arg⁸-Pro⁹-D-Ala¹⁰-NH₂.

4. Composé selon la revendication 1 répondant à la formule :
Ac-D-Nal(2)¹-D-Cpa²-D-Pal(3)³-Ser⁴-N-Me-Tyr⁵-D-HCl⁶-Nle⁷-Lys(lPr)⁸-Pro⁹-Ala¹⁰-NH₂.

5. Composé selon la revendication 1 répondant à la formule :
Ac-D-Nal(2)¹-D-Cpa²-D-Pal(3)³-Ser⁴-N-Me-Tyr⁵-D-HCi⁶-Nle⁷-Lys(iPr)⁸-Pro⁹-Sar¹⁰-NH₂.

6. Composé selon la revendication 1 répondant à la formule :
Ac-D-Nal(2)¹-D-Cpa²-D-Pal(3)³-Ser⁴-N-Me-Tyr⁵-D-HCi⁶-Nle⁷-Arg⁸-Pro⁹-Sar¹⁰-NH₂.

7. Composition pharmaceutique comprenant un composé selon l'une quelconque des revendications 1 à 6.

8. Procédé pour la préparation de composés répondant à la formule générale I, selon la revendication 1, dans lequel des fragments d'éléments constitutifs Xxx^{m} pourvus de groupes protecteurs appropriés, dans lesquels m représente un nombre entier de 1 à 10 et Xxx¹ est acétylé, sont construits sur une phase solide ou en solution selon des procédés courants, ensuite les fragments sont joints par accouplement de segments sur une phase solide et, une fois l'accouplement terminé, les composés répondant à la formule générale I sont séparés avec des procédés courants avec amidation sur l'élément constitutif Xxx¹⁰, de la phase solide.

9. Utilisation des substances selon les revendications 1 à 6 pour la fabrication de médicaments pour le traitement des tumeurs dépendant d'hormones, en particulier le carcinome de la prostate ou le cancer du sein, ainsi que pour des indications non malignes dont le traitement exige une suppression d'hormone - LH- RH.

10. Procédé pour la fabrication de médicaments, dans lequel on mélange des composés selon les revendications 1 à 6 avec les supports et adjuvants courants et on les confectionne sous forme de médicaments.
